# EUROPEAN PATENT APPLICATION

(11) **EP 3 225 624 A1**
(43) Date of publication of application: **04.10.2017**
(21) Application number: 15863556.5
(22) Date of filing: 27.11.2015
(51) Int. Cl.: C07D 487/14, H01L 51/42

(54) **NOVEL COMPOUND AND USE THEREOF AS A HOLE-TRANSPORT MATERIAL**

(30) Priority: 28.11.2014 ES 201431776
(71) Applicant: Abengoa Research, S.L., 41014 Sevilla (ES)
(72) Inventor: AHMAD, Shahzada, 41014 Sevilla (ES); RAMOS, Francisco Javier, 41014 Sevilla (ES); KAZIM, Samrana, 41014 Sevilla (ES); DOBLARÉ CASTELLANO, Manuel, 41014 Sevilla (ES); NAZEERUDDIN, Mohammad Khaja, 1015 Lausanne (CH); GRAETZEL, Michael, 1015 Lausanne (CH); RAKSTYS, Kasparas, 1015 Lausanne (CH)
(74) Representative: Balder IP Law, S.L.
(86) International application number: PCT/ES2015/070864
(87) International publication number: WO 2016/083655

(57) **Abstract**

The present invention provides novel triazatruxene derivatives that are useful as hole transport materials (HTM), particularly, in optoelectronic devices. The utility of the novel compounds was confirmed in solid-state, sensitized solar cells based on organic-inorganic perovskites used as light harvesters. The devices achieved high power conversion efficiencies.

## Description

### Technical Field

The present invention relates to novel compounds, methods for preparing the compounds, methods and uses of the compounds as hole transport material, in optoelectronic and/or electrochemical devices comprising the compounds, and methods for producing the optoelectronic and/or electrochemical devices.

### Prior Art and the Problem Underlying the Invention

The conversion of solar energy to electrical current using thin film in third generation photovoltaics (PV) is being widely explored for the last two decades. The sandwich/monolithic-type PV devices, consisting of a mesoporous photoanode with an organic/inorganic light harvester, redox electrolyte/solid-state hole conductor, and counter electrode, have gained significant interest due to the ease of fabrication, flexibility in the selection of materials and cost effective production (Grätzel, Acc. Chem. Res. 2009, 42, 1788-1798; Hagfeldt et al., Chem. Rev. 2010, 110, 6595-6663). Recently, bulk layers of organometallic halide perovskite based on tin (CsSnX₃, Chung et al., Nature. 2012, 485, 486-489) or lead (CH₃NH₃PbX₃, Kojima et al., J. Am. Chem. Soc. 2009, 131, 6050-6051; Etgar et al., J. Am. Chem. Soc. 2012, 134, 17396-17399; Kim et al., Sci. Rep. 2012, 2, 591:1-7; Lee et al., Science 2012, 338, 643-647) have been introduced as semiconducting pigment for light harvesting, resulting in high power conversion efficiencies (PCE).

Currently most performing solid state device, doped Spiro-OMeTAD (2,2',7,7'-tetrakis(N,N-di-p-methoxyphenyl amine)-9,9-spirobifluorene) is used as a hole transport material (HTM) for transporting holes from the working electrode, formed by the semiconductor and light harvester, to the cathode, thereby closing the electric circuit of the operating cell. The relatively low PCE of solid state devices was often ascribed to the low hole mobility in Spiro-OMeTAD, which causes interfacial recombination losses by two orders of magnitude higher than in liquid, dye-sensitized solar cells (DSCCs).

Attempts were made to find an alternate organic HTM having higher charge carrier mobility and matching HOMO level to replace Spiro-OMeTAD. In most of the cases, it is difficult to compete with the performances equivalent to Spiro-OMeTAD-based devices, generally due to incomplete pore filling.

The ideal conditions to be fulfilled by HTM in order to give good PV performance are sufficient hole mobility, thermal and UV stability, and well-matched HOMO (highest occupied molecular orbital) energy level to the semiconductor light absorbers.

Poly[N-9-heptadecanyl-2,7-carbazole-alt-3,6-bis-(thiophen-5-yl)-2,5-dioctyl-2,5-dihydropyrrolo[3,4-]pyrrole-1,4-dione] (PCBTDPP) as HTM has been introduced in perovskites based cells. These devices were made in a configuration using mesoporous (mp), mp-TiO₂/CH₃NH₃PbBr₃/PCBTDPP/Au. The CH₃NH₃PbBr₃ cells showed PCE of 3.0% with open circuit voltage (V_{oc}) of 1.15 eV. Poly(3-hexylthiophene), poly-[2,1,3-benzothiadiazole-4,7-diyl[4,4-bis(2-ethylhexyl)-4H-cyclopenta[2,1-b:3,4b]dithiophene-2,6-diyl]] (PCPDTBT), poly-[[9-(1-octylnonyl)-9H-carbazole-2,7-diyl]-2,5-thiophenediyl-2,1,3-benzothiadiazole-4,7-diyl-2,5- thiophenediyl] (PCDTBT), and poly(triarylamine) (PTAA) were used as HTM together with perovskites (CH₃NH₃PbI₃) as light harvester. Due to its polymeric nature it has large chains, thus will induce defects and low device reproducibility, furthermore polymers are known to be unstable in the low vacuum conditions that follow the step of depositing a cathode.

In these devices, the low fill factor (FF) could be due to a trade-off between series and shunt resistance. Thus, one may envisage increasing the *FF* by making pin hole free thin layers of perovskite and exploiting the synergy with new HTMs having relatively low series resistance.

Due to the highly conductive nature of perovskite, a thick layer of HTM is required to avoid pinholes. On the other hand, this thicker HTM overlayer increases series resistance due to its less conductive nature.

In brief, an objective of the invention is to provide a HTM, which is easily available, cost effective and resulting in solar cells having good PCE, in a solid-state configuration. HTM which is free from additive or dopant is also critical for long term stability.

The present invention addresses the problems depicted above.

### Summary of the Invention

Remarkably, the present inventors identified novel candidates of compounds that are useful as HTMs for optoelectronic and/or electrochemical devices, such as solid state solar cells.

In an aspect, the present invention provides compounds comprising the structure of formulae (I) below: 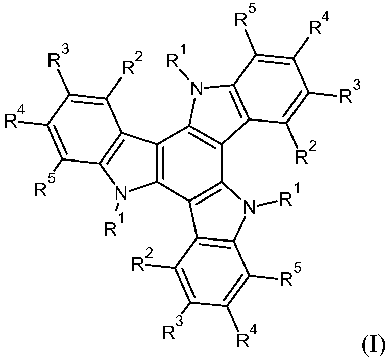 wherein R¹ is selected from substituted or unsubstituted alkyl, alkenyl, alkynyl, and aryl, and wherein R²-R⁵, are selected independently from H, substituted or unsubstituted alkyl, alkenyl, alkynyl, aryl, and substituents of formula (II), (III) and (IV) below, 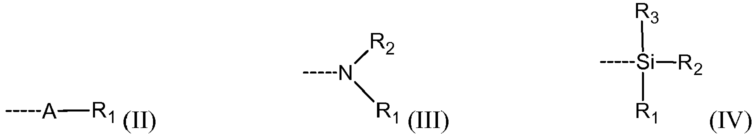 wherein A is selected from O, S, or Se, and from other electron donor moiety, and R₁, R₂, and R₃ are independently selected from alkyl, alkenyl, alkynyl, aryl;
wherein any one of said alkyl, alkenyl, and alkynyl may be linear, branched or cyclic.

In an aspect the present invention provides soluble derivatives of triazatruxene and/or of 10,15-dihydro-5H-diindolo[3,2-a:3',2'-c]carbazole.

In an aspect the present invention provides soluble compounds selected from 5,10,15-trihexyl-3,8,13-trimethoxy-10,15-dihydro-5H-diindolo[3,2-a:3',2'-c]carbazole (HMDI) and 5,10,15-tris(4-(hexyloxy)phenyl)-10,15-dihydro-5H-diindolo[3,2-a:3',2'-c]carbazole (HPDI).

In an aspect, the present invention provides an optoelectronic and/or electrochemical device comprising a compound of the present invention.

In an aspect, the present invention provides an optoelectronic and/or electrochemical device comprising soluble derivatives of triazatruxene and/or of 10,15-dihydro-5H-diindolo[3,2-a:3',2'-c]carbazole.

In an aspect, the present invention provides solar cells, in particular perovskite-based solar cells comprising a compound of the invention.

In an aspect, the present invention provides the use of the compounds of the invention as HTM.

In an aspect, the present invention provides the use of the compounds of the invention as HTM in a solar cell, in particular a perovskite-based solar cell.

In an aspect, the present invention provides the use of the compounds of the invention as HTM in a solar cell where dopants are absent in the HTM.

In an aspect, the present invention provides the use of the compounds of the invention as HTM in a solar cell where the HTM comprises less than 20ml.% of dopant.

In an aspect, the present invention comprises a method for providing a HTM, the method comprises the step of providing the compounds of the present invention.

In an aspect, the present invention provides a process for producing a solar cell comprising the steps of applying a plurality of layers comprising at least one HTM and further layers as necessary so as to provide said solar cell, wherein said hole transport layer comprises a compound selected from the compounds of the present invention.

In an aspect, the present invention provides a process for producing a solar cell comprising the steps of applying a plurality of layers comprising an organic-inorganic perovskite layer, a hole transport layer and a conducting current providing layer, wherein said hole transport layer comprises an HTM comprising a compound selected from the compounds of the invention.

In an aspect, the present invention provides a method for preparing the compounds of the invention, the method comprising the steps of: providing triazatruxene, and, substituting hydrogens thereof so as to provide the compounds of the invention.

Further aspects and preferred embodiments of the invention are defined herein below and in the appended claims. Further features and advantages of the invention will become apparent to the skilled person from the description of the preferred embodiments given below.

The novel compounds of the invention provide several, important advantages. The new compounds are solution processable and can be easily coated by various techniques, methods, such as dip-, spin- or spray-coating or can be printed. The novel compounds have a good solubility in non polar organic solvents, which allows the use of a wide choice of solvents for preparing devices containing the compounds. They are easy to synthesize, thermally stable up to 350 °C and transparent in the visible part of the solar spectrum.

These new compounds were shown to have good charge (hole) transport properties in their pristine form, which resulted in better PV properties. Interestingly, devices containing undoped HTMs performs generally better or at least similar like doped HTMs. Device performance was better than that achieved with the prior state of the art material of choice, (undoped) Spiro-OMeTAD.

### Brief Description of the Drawings

**Figure 1** is a scheme illustrating the synthesis of exemplary compounds in accordance with preferred embodiments of the invention.
**Figure 2** shows J-V characteristics of a solar cell containing an HTM compounds HMDI and HPDI according to embodiments of the invention as shown in Example 1.
**Figure 3** shows incident photon-to-electron conversion efficiencies (IPCE) for a compound (HMDI and HPDI) according to an embodiment of the invention, as hole transporter in a mesoscopic perovskite solar cells.
**Figure 4** shows cyclic voltammograms of compounds according to embodiments of the invention, referred to as HMDI and HPDI molecules, established in a three electrode cell.
**Figure 5** shows UV-Vis absorption spectra of exemplary HMDI and HPDI molecules in chlorobenzene.
**Figure 6** shows a thermogravimetric analysis of exemplary compounds HMDI and HPDI. It can be seen that the compounds are stable up to temperatures of around 350°C.
**Figures 7** and **8** show exemplary device structures of the optoelectronic and/or electrochemical devices of the invention.
**Figures 9** **A** and **B** show various exemplary compounds in accordance with embodiments of the invention.
**Figure 10** shows the J-V curve of a photovoltaic solar cell that contains S4 compound compared to spiro-OMeTAD, in accordance with embodiments of the invention as it is shown in Example 2. Curves were recorded scanning at 0,01 V s⁻¹ from forward bias (FB) to short circuit (SC) condition and vice versa. The prepared HTM (S4) showed a lower hysteresis value.

### Detailed Description of the Preferred Embodiments

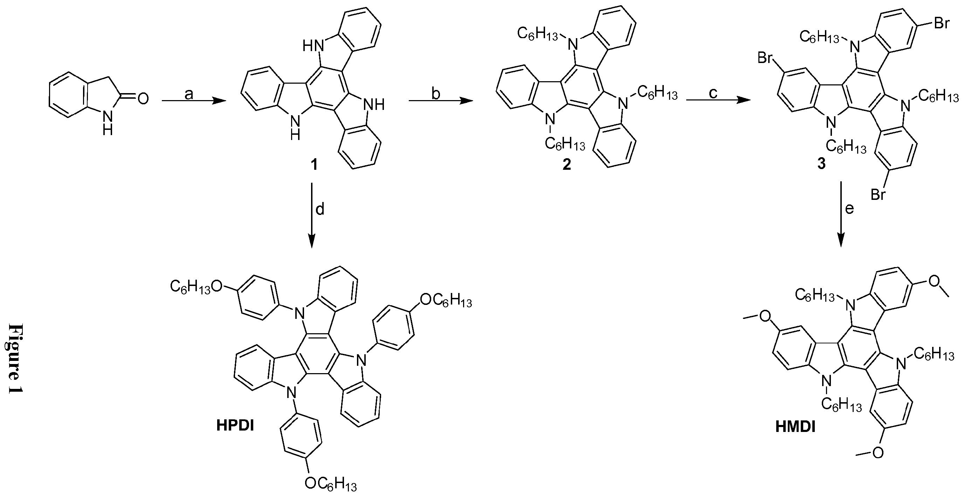

In an aspect, the present invention provides novel compounds of formula (I). These compounds are preferably derivatives of 10,15-dihydro-5H-diindolo[3,2-a:3',2'-c]carbazole (compound (1) in Figure 1). For the purpose of the present specification the trivial name triazatruxene is used as equivalent of 10,15-dihydro-5H-diindolo[3,2-a:3',2'-c]carbazole.

For the purpose of the present specification, the expression "comprise" and its various grammatical forms, such as "comprising", etc., is intended to mean "includes, amongst other". It is not intended to mean "consists only of".

In said compounds, substituent R¹ is preferably selected from substituted or unsubstituted alkyl, alkenyl, alkynyl, and aryl. Any one or more of said alkyl, alkenyl, and alkynyl may be linear, branched or cyclic.

In an embodiment, R¹ preferably comprises altogether (including optional substituents of said alkyl, alkenyl, alkynyl, and aryl) from 1-20 carbons and from 0-10 heteroatoms, preferably 3-15 carbons and 0-5 heteroatoms, most preferably 4-12 carbons and 0-3 heteroatoms.

Substituents of said alkyl, alkenyl, alkynyl, (if substituted) may be selected from aryl, alkylaryl, alkoxylaryl, halogen, and substituents of any one of formulae (II) to (IV).

Substituents of said aryl (if substituted) may be selected from alkyl, alkenyl, alkynyl, halogen, and from substituents of any one of formulae (II) to (IV).

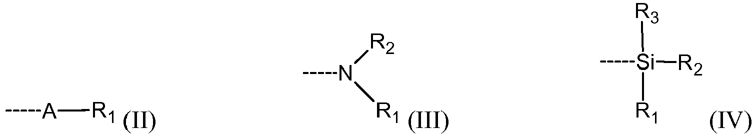

In said substituents of formulae (II)-(IV), R₁, R₂, and R₃, in as far as present, are independently selected from alkyl, alkenyl, alkynyl, aryl. In said substituents of formulae (II), A is selected from O, S, or Se or another suitable electron donor moiety.

Preferred optional substituents of said alkyl, alkenyl, alkynyl, and aryl in R¹ are substituents of formula (II), more preferably substituents of formula (II) in which A is O and R¹ is alkyl, for example C1-C12 alkyl, preferably C4-C10 alkyl, and more prefereably C6.

In the compounds of formula (I), R²-R⁵ can be selected independently from H, substituted or unsubstituted alkyl, alkenyl, alkynyl, aryl, and substituents of formula (II), (III) and (IV) (see above), wherein any one of said alkyl, alkenyl, and alkynyl may be linear, branched or cyclic.

Altogether (including optional substituents of said alkyl, alkenyl, alkynyl, and aryl), if different from H, R²-R⁵ preferably comprise, independently, from 1-20 carbons and from 1-10 heteroatoms, preferably 1-15 carbons and 1-5 heteroatoms, even more preferably 1-12 carbons and 0-3 heteroatoms, most preferably 1-6 carbons and 1 heteroatom.

In an embodiment, R²-R⁵ are independently selected from H, unsubstituted alkyl, alkenyl, alkynyl, aryl, and substituents of formula (II), (III) and (IV) (see above), wherein any one of said alkyl, alkenyl, and alkynyl may be linear, branched or cyclic. Preferably, R²-R⁵ are independently selected from H and from substituents of formula (II). Preferably, in said substituent (II), A is O. Preferably, R₁ is independently selected from C1-C20 alkyl, preferably C1-C12 alkyl, most preferably C1-C8 alkyl.

In a preferred embodiment, one or more of R²-R⁵ is/are different from H and thus as defined in accordance with the embodiments of R²-R⁵ as defined in this specification.

In a preferred embodiment, said one or more substituents of R²-R⁵ that is/are different from H is/are alkoxy, preferably C1-C12 alkoxy, more preferably C1-C8 alkoxy.

In an embodiment of the compounds of the invention, R¹ is selected from alkyl and substituted aryl, wherein substituents of said aryl are selected from alkyl and from substituents of formula (II), (III) or (IV), and wherein R²-R⁵, are selected independently from H, alkyl, and substituents of formula (II), (III) or (IV). Preferably, one or more of R²-R⁵ is/are different from H.

R¹ preferably comprises an alkyl, for example in the following possibilities: (i) R¹ is alkyl; (ii) R¹ is an alkyl-substituted aryl, or (iii) R¹ is an aryl substituted with a substituent of formulae (II)-(IV), preferably of formula (II), wherein R₁-R₃ are selected independently from alkyl. Preferably, said alkyl (in R¹) is a C1-C20 alkyl, preferably a C2-C15 alkyl, most preferably a C4-C12 alkyl, in particular an alkyl selected from C6, C8 or C10 alkyls. Preferably, said alkyl contained in R¹ is a linear alkyl.

On the other hand, in said substituent R²-R⁵, if they are different from H, they preferably also comprise an alkyl, for example in the following possibilities: (i) at least one of R²-R⁵ is alkyl; (ii) at least one of R²-R⁵ is a substituent of formulae (II)-(IV), preferably of formula (II), wherein R₁-R₃ are selected independently from alkyl. Preferably, said alkyl is a C1-C12 alkyl, more preferably a C1-C8 alkyl, most preferably a C1-C6 alkyl, for example a C1 alkyl.

In a preferred embodiment, R¹ is selected from alkyl and substituted phenyl, wherein substituents of said phenyl are selected, independently, from alkyl and from alkoxyl, and wherein R²-R⁵ are selected, independently, from H, alkyl, and alkoxyl, more preferably from H and alkoxyl. Preferably, one or more substituents of R²-R⁵ is/are different from H (and thus are, independently, an alkyl or alkoxyl). Preferably, with respect to the sizes (number of carbons) of said alkyls substituents in R¹ and R²-R⁵, including alkyl part of alkoxyls, the same as said above applies.

In an embodiment, the compound of the invention is selected from compounds of formula (V) below: 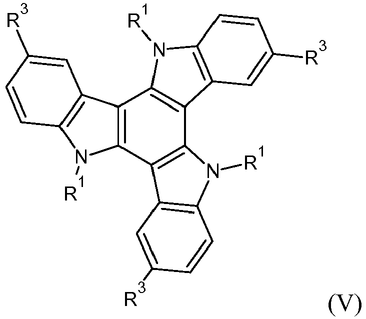 wherein R¹ and R₃ are as defined with respect to the present invention, preferably as defined with respect to embodiments and preferred embodiments specified above. The present embodiment differs from previous embodiments in that it is specified that R², R⁴ and R⁵ are always H and R₃ is selected from H and from substituents that are different from H as defined elsewhere in this specification. In particular, R³ may be selected from substituted or unsubstituted alkyl, alkenyl, alkynyl, aryl, and substituents of formula (II), (III) and (IV) (see above), wherein any one of said alkyl, alkenyl, and alkynyl may be linear, branched or cyclic.

In a preferred embodiment, one or more of R²-R⁵ is/are different from H and thus as defined in accordance with the embodiments of R²-R⁵ as defined in this specification.For example, in an embodiment, R¹ is selected from alkyl and substituted aryl, wherein substituents of said aryl are selected from alkyl and from substituents of formula (II), (III) or (IV), preferably of formula (II), and wherein R³ is selected independently from H, alkyl, and substituents of formula (II), (III) or (IV), preferably of formula (II). In another preferred embodiment, R¹ is selected from alkyl and substituted phenyl, wherein substituents of said phenyl are selected, independently, from alkyl and from alkoxyl, and wherein R³ is selected, independently, from H, alkyl, and alkoxyl, more preferably from H and alkoxyls. For these embodiments, the same as indicated above applies, for example with respect to the presence and size of alkyls.

In a preferred embodiment, the compound of the invention is selected from compounds of formula (VI) and (VII) below: 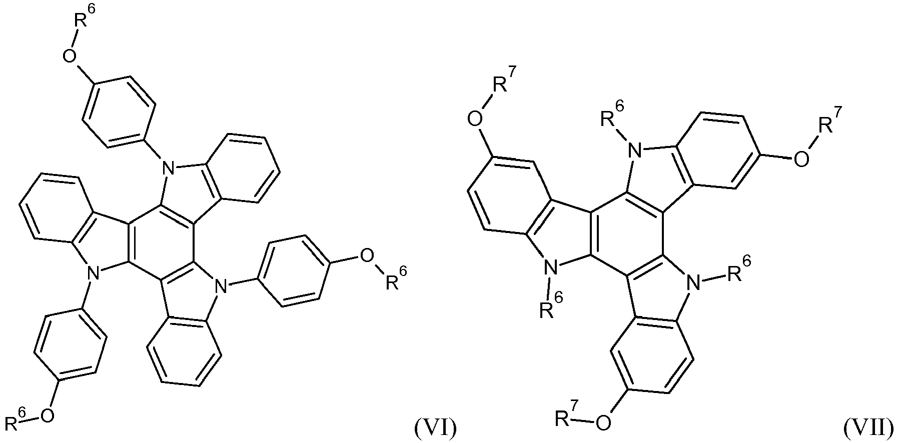 wherein R⁶ and R⁷ are defined, independently, as substituent R₁ as defined elsewhere in this specification. Preferably, R⁶ and R⁷ are independently selected from linear, branched or cyclic C1-C15 alkyls. In a preferred embodiment, R⁶ and R⁷ are independently selected from linear, branched or cyclic C1-C12 alkyls. In a more preferred embodiment, R⁶ is selected from linear and branched C4-C10 alkyls, preferably C4-C8 alkyls, and R⁷ is selected from linear and branched C1-C10, preferably C1-C4 and most preferably C1 alkyls.

In a preferred embodiment, the present invention provides soluble derivatives of triazatruxene. In an embodiment, the compounds of the invention are soluble in one or more of the solvents selected from chlorobenzene, benzene, 1,2-dichlorobenzene and toluene. Preferably, the compounds are soluble at least in toluene. For example, the compounds are soluble in any one, several or all of these solvents. In a preferred embodiment, the compounds are soluble in all the four aforementioned solvents. The presence of solubility is preferably determined at room temperature (25°C) under stirring for up to 10 minutes.

Preferably, the compounds of the invention are soluble at least and/or more than 20mg, preferably 50 mg of compound per ml of solvent. More preferably, the compounds are soluble at least and/or more than 100 mg of compound per ml of solvent, preferably 150 mg of compound per ml of solvent.

In a preferred embodiment, the compounds of the invention are soluble at least and/or more than 200 mg of compound per ml of toluene solvent.

In a preferred embodiment, the compounds of the invention are selected from 5,10,15-trihexyl-3,8,13-trimethoxy-10,15-dihydro-5H-diindolo[3,2-a:3',2'-c]carbazole (compound (HMDI) in Fig. 1) and 5,10,15-tris(4-(hexyloxy)phenyl)-10,15-dihydro-5H-diindolo[3,2-a:3',2'-c]carbazole (compound (HPDI) in Fig. 1).

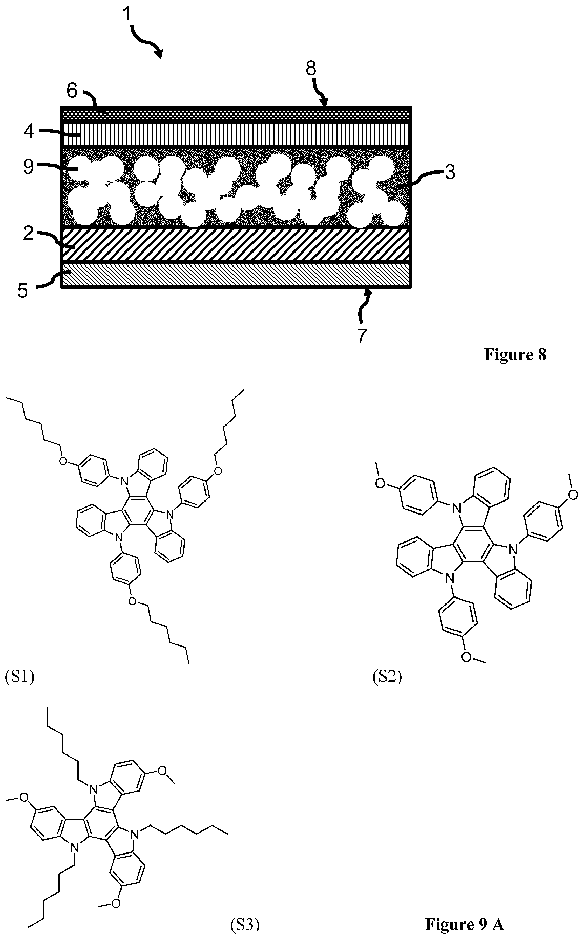

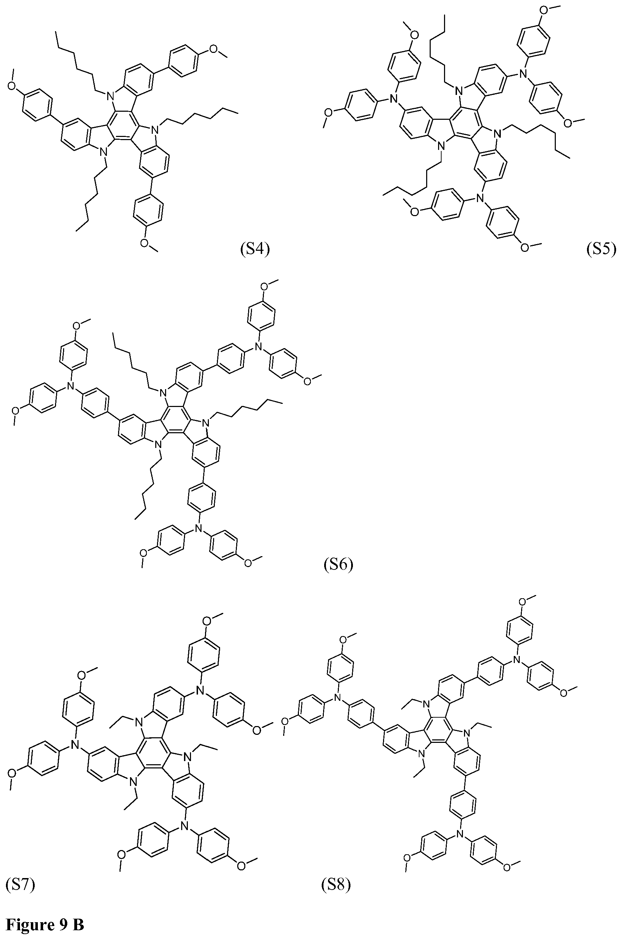

**Figures 9A** and **B** show other exemplary compounds ((S1)-(S8)) in accordance with the invention. These compounds are: (S1): 5,10,15-tris(4-(hexyloxy)phenyl)-10,15-dihydro-5H-diindolo[3,2-a:3',2'-c]carbazole; (S2): 5,10,15-tris(4-methoxyphenyl)-10,15-dihydro-5H-diindolo[3,2-a:3',2'-c]carbazole; (S3): 5,10,15-trihexyl-3,8,13-trimethoxy-10,15-dihydro-5H-diindolo[3,2-a:3',2'-c]carbazole; (S4): 5,10,15-trihexyl-3,8,13-tris(4-methoxyphenyl)-10,15-dihydro-5H-diindolo[3,2-a:3',2'-c]carbazole; (S5): 5,10,15-trihexyl-N³,N³,N⁸,N⁸,N¹³,N¹³-hexakis(4-methoxyphenyl)-10,15-dihydro-5H-diindolo[3,2-a:3',2'-c]carbazole-3,8,13-triamine, (S6): 4,4',4"-(5,10,15-trihexyl-10,15-dihydro-5H-diindolo[3,2-a:3',2'-c]carbazole-3,8,13-triyl)tris(N,N-bis(4-methoxyphenyl)aniline), (S7): 5,10,15-triethyl-N³,N³,N⁸,N⁸,N¹³,N¹³-hexakis(4-methoxyphenyl)-10,15-dihydro-5H-diindolo[3,2-a:3',2'-c]carbazole-3,8,13-triamine; (S8): 4,4',4"-(5,10,15-triethyl-10,15-dihydro-5H-diindolo[3,2-a:3',2'-c]carbazole-3,8,13-triyl)tris(N,N-bis(4-methoxyphenyl)aniline).

In another embodiment, the compound of the invention is selected from the compounds (S9)-(S17) below, based on the structure of formula (VIII): 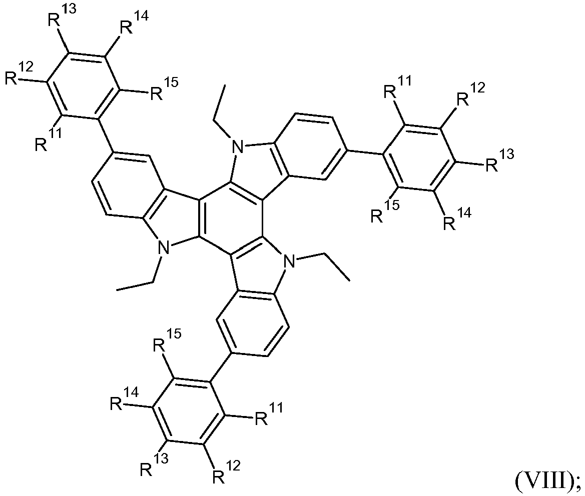 wherein R¹¹-R¹⁵ in compounds (S9)-(S17) are selected as follows:
Compound (S9): R¹¹=OMe, R¹²=H, R¹³=H, R¹⁴=H, R¹⁵=H.
Compound (S10): R¹¹=H, R¹²=OMe, R¹³=H, R¹⁴=H, R¹⁵=H.
Compound (S11): R¹¹=H, R¹²=H, R¹³=OMe, R¹⁴=H, R¹⁵=H.
Compound (S12): R¹¹=OMe, R¹²=OMe, R¹³=H, R¹⁴=H, R¹⁵=H.
Compound (S13): R¹¹=OMe, R¹²=H, R¹³=OMe, R¹⁴=H, R¹⁵=H.
Compound (S14): R¹¹=H, R¹²=OMe, R¹³=OMe, R¹⁴=H, R¹⁵=H.
Compound (S15): R¹¹=OMe, R¹²= OMe, R¹³=OMe, R¹⁴=H, R¹⁵=H.
Compound (S16): R¹¹=OMe, R¹²= H, R¹³=OMe, R¹⁴=H, R¹⁵=OMe.
Compound (S17): R¹¹=H, R¹²=OMe, R¹³=OMe, R¹⁴=OMe, R¹⁵=H.

Surprisingly, the compounds of the invention are advantageous organic HTMs. The compounds are particular advantageous as HTM in optoelectronic devices, such as sensitized solar cells. In preferred embodiment, the invention provides an organic-inorganic perovskite based solar cell comprising the compound of the present invention. In the optoelectronic devices of the invention, in particularly in the solar cells, the compound of the invention is preferably provided as HTM in the HTM layer of said devices.

In preferred embodiments, the optoelectronic devices, in particular solar cells, are preferably flat devices when considered on a macroscopic scale. According to a preferred embodiment, they are layered and/or comprise and/or consist essentially of a plurality of layers. In view of their flat configuration, the devices of the invention preferably have two opposing sides, a first side and a second side, said opposing sides preferably making up the majority of the macroscopic surface of the device of the invention.

The compounds of the invention are particularly advantageous as HTM as hole transporter in perovskite-based solar cells. These devices are generally based on the architecture of "dye-sensitized solar cells", often abbreviated as DSSCs or DSC, in layers of organometallic halide perovskites are used instead of organic dyes or metal-complex-based dyes.

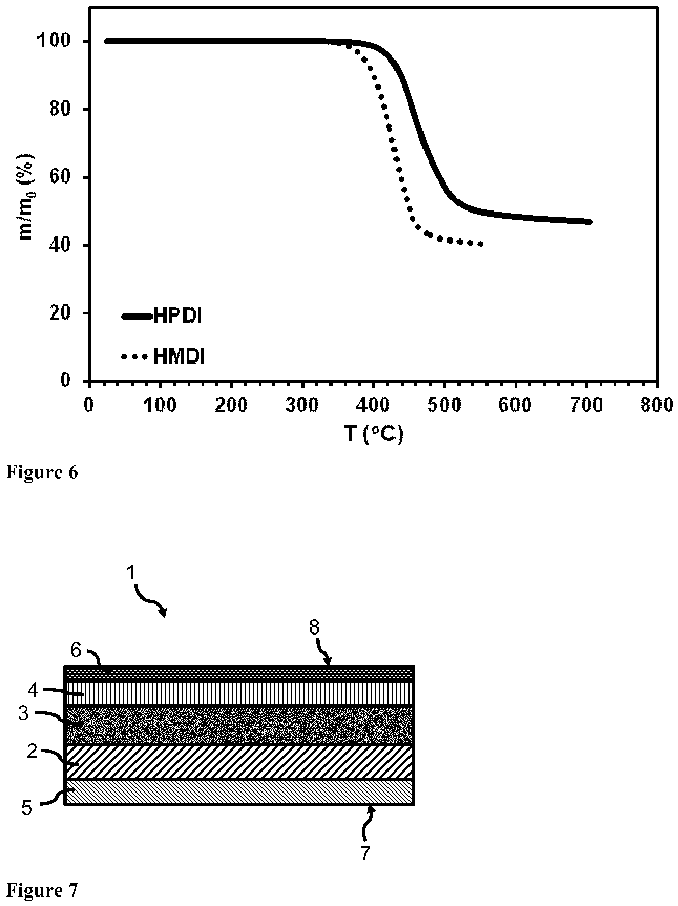

In an embodiment, the invention provides a solar cell 1 as illustrated in **Figure 7****.** The solar cell comprises two opposing sides 7, 8, which may be (arbitrarily) referred to as a first side 7 and a second side 8. The solar cell in accordance with this embodiment preferably comprises a conducting current collector layer 5, an n-type semiconductor layer 2, an organic-inorganic perovskite layer 3, a hole transport layer 4 and a conducting current providing layer 6, wherein said hole transport layer 4 is provided between said perovskite layer 3 and said current providing layer 6, said hole transport layer comprising a compound selected from the compounds of the invention.

In the solar cells of the invention, the HTM layer 4, which comprises the compounds of the invention, has preferably a thickness of 50-400 nm, preferably 100-200 nm, even more preferably 110-190 nm or 120-180 nm, and most preferably 100-170 nm for example about 150 nm. Interestingly, in the devices of the present invention, the HTM layer may be less thick than devices reported in the prior art, in which another HTM is used.

The advantage of using the thin layer of HTM is first to utilize less material and secondly to make an equilibrium between series resistance and shunt resistance. The new HTM of the invention advantageously allows choosing a comparatively low thickness while still avoiding short circuits,

In an embodiment, the HTM layer in the device of the invention, in particular in the solar cell, for example as shown in Figs 7 or 8, comprises less than 20 % of a dopant, wherein said percentage represents the molar ratio of dopant compounds with respect to the said HTM compound (mol/mol). Surprisingly, the presence of conventional dopants commonly used with Spiro-OMeTAD, did not have an important impact on the performance of the devices of the invention containing the novel compounds of the invention. In a preferred embodiment, the HTM layer in the device of the invention comprises less than 15 %, less than 10%, less than 5%, less than 3%, less than 1% of a dopant (mol/mol) or is dopant free. The fact that dopants are not needed or are not mandatory represents an important advantage. First, fewer components are needed for device fabrication, which also reduces the number of steps for fabricating the device. Secondly the addition of dopants make them hygroscopic and induce defects, which will result in reduction in long term efficiency.

In an embodiment, said doping compound, which is preferably absent or preferably present at molar ratios as indicated above is lithium bis-(trifluoromethylsulfonyl)imide (LiTFSI). In a more preferred embodiment, LiTFSI taken together with the additive ter Butylpyridine (t-BP), are either absent or present independently at molar ratios indicated above. For example, in the HTM layer of the device, the combined molar concentration of t-BP and LiTFSI is lower than 20% of the molar concentration of the novel HTM compounds of the invention.

In particular in devices based on the exemplary compound HPDI, the presence of dopants, in particular LiTFSI combined with the additive t-BP, had a negative influence of the power conversion efficiency (η) of the devices.

Other dopants that are frequently used in HTMs are FK 209: tris(2-(1H-pyrazol-1-yl)-4-tert-butylpyridine)cobalt(III) tris- (bis(trifluoromethylsulfonyl)imide); H-TFSI: Hydrogen bis(trifluoromethanesulfonyl)imide; FK269: bis(2,6- di(1H-pyrazol-1-yl)pyridine)cobalt(III) tris(bis(trifluoromethylsulfonyl)-imide)); FK102: tris(1-(pyridin-2-yl)-1H-pyrazol) cobalt(III)tris-(hexafluorophosphate); and F4TCNQ: perfluoro-tetracyano-quinodimethane;

Other additive that can be used in HTMs is 2,6-Dimethylpyridine.

Surprisingly, the presence of other dopants or LiTFSI with the additive t-BP in combination with other dopants in the HTM did not result in an improvement of the PV properties. Therefore, in a preferred embodiment, all dopants present in the HTM, in particular the dopants and other dopants specified above, are present at a molar concentration of ≤ 20mol%, preferably ≤ 15mol%, and most preferably 10 <mol%.

In the method for preparing a solar cell in accordance with the invention, the HTM layer may be applied by one selected from the group of: spin-coating, dip-coating, spray-coting, sublimation, printing, slot die coating or any other coating techniques.

The organic inorganic perovskite layer 3 is preferably provided between the n-type semiconductor layer 2 and the hole transport layer 4. In the absence of an optional blocking layer, the perovskite layer 3 is preferably in direct contact with the n-type semiconductor layer 2 on one side and with the hole transport layer 4 on the other side.

In principle, any suitable organic inorganic perovskite may be used for layer 4. Such organic inorganic perovskites that are useful for solar cells have been disclosed in the literature.

Preferred organic inorganic perovskites are disclosed, for example, in the international application WO 2014/020499, filed on July 24, 2013. The organic-inorganic perovskites disclosed in this application are expressly incorporated herein by reference. More specifically, the organic inorganic perovskite may be selected, for example, from the compounds disclosed from page 10, line 30, through page 17, line 21. This disclosure is expressly incorporated herein by reference.

Furthermore, organic-inorganic perovskites may be selected from those disclosed in international application WO 2013/171517, filed on May 20, 2013, which discloses in particular mixed-anion perovskites, which may also be used for the purpose of the present invention, in particular as disclosed from page 8, the paragraph starting with "The term "perovskite" as defined herein ...", through page 19, including the first paragraph on page 19. This disclosure is expressly incorporated herein by reference.

According to a preferred embodiment, said organic-inorganic perovskite comprises a perovskite structure selected of any one of the formulae (XX) to (XXV) below;

APbX₃ (XX)

ASnX₃ (XXI)

A₂PbX₄ (XXII)

A₂SnX₄ (XXIII)

BPbX₄ (XXIV)

BSnX₄ (XXV)

wherein:
A is an organic, monovalent cation selected from primary, secondary, tertiary or quaternary organic ammonium compounds, including N-containing heterorings and ring systems, A having from 1 to 15 carbons and 1 to 10 heteroatoms;
B is an organic, bivalent cation selected from primary, secondary, tertiary or quaternary organic ammonium compounds having from 1 to 15 carbons and 2-10 heteroatoms and having two positively charged nitrogen atoms;
the three or four X are independently selected from Cl⁻, Br⁻, I⁻, NCS⁻, CN⁻, and NCO⁻, preferably from Br⁻ and I⁻.

In an embodiment, the organic-inorganic perovskite is free of Pb. Accordingly, in some embodiments, the metal atom is different from Pb. The metal atoms can be Sn, as shown in formulae (XXI), (XXIII), and (XXV), but other metals may also be used to replace Sn. For example, Pb in formulae (XX), (XXII) and (XXIV) may be replaced by any one or more selected from Cu²⁺, Ni²⁺, Co²⁺, Fe²⁺, Mn²⁺, Cr²⁺, Pd²⁺, Zn²⁺, Cd²⁺, Ge²⁺, Sn²⁺, Pb²⁺, Eu²⁺, and Yb²⁺.

Preferably, A in particular in any one of formulae (XX) to (XXIII), is a monovalent cation selected from any one of the compounds of formulae (1) to (8) below: 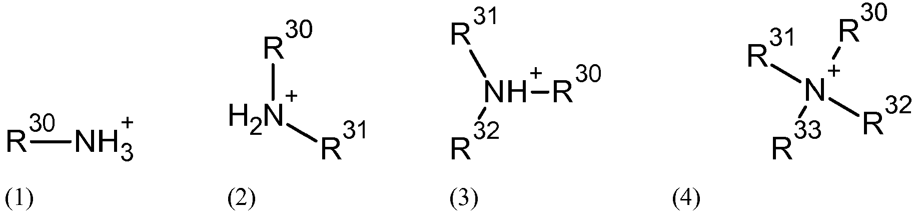 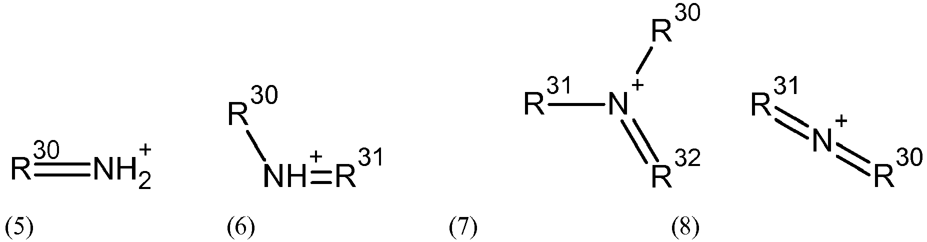 wherein,
any one of R³⁰, R³¹, R³² and R³³, in as far as present, is independently selected from C1 to C15 aliphatic and C4 to C15 aromatic substituents, wherein any one, several or all hydrogens in said substituent may be replaced by halogen.

According to an embodiment, any one of R³⁰, R³¹, R³² and R³³, as far as present, is independently selected from C1 to C8 aliphatic and C4 to C8 aromatic substituents wherein any one, several or all hydrogens in said substituent may be replaced by halogen.

According to an embodiment, any one of R³⁰, R³¹, R³² and R³³, as far as present, is independently selected from C1 to C4, preferably C1 to C3 and most preferably C1 to C2 aliphatic substituents wherein any one, several or all hydrogens in said substituent may be replaced by halogen.

According to an embodiment, any one of R³⁰, R³¹, R³² and R³³, as far as present, is independently selected from C1 to C8 alkyl, C2 to C8 alkenyl and C2 to C8 alkynyl, wherein said alkyl, alkenyl and alkynyl, if they comprise 3 or more carbons, may be linear, branched or cyclic, and wherein several or all hydrogens in said substituent may be replaced by halogen.

According to an embodiment, any one of R³⁰, R³¹, R³² and R³³, in as far as present, is independently selected from C1 to C6 alkyl, C2 to C6 alkenyl and C2 to C6 alkynyl, wherein said alkyl, alkenyl and alkynyl, if they comprise 3 or more carbons, may be linear, branched or cyclic, and wherein several or all hydrogens in said substituent may be replaced by halogen.

According to an embodiment, any one of R³⁰, R³¹, R³² and R³³, as far as present, is independently selected from C1 to C4 alkyl, C2 to C4 alkenyl and C2 to C4 alkynyl, wherein said alkyl, alkenyl and alkynyl, if they comprise 3 or more carbons, may be linear, branched or cyclic, and wherein several or all hydrogens in said substituent may be replaced by halogen.

According to an embodiment, any one of R³⁰, R³¹, R³² and R³³, in as far as present, is independently selected from C1 to C3, preferably C1 to C2 alkyl, C2 to C3, preferably C2 alkenyl and C2 to C3, preferably C2 alkynyl, wherein said alkyl, alkenyl and alkynyl, if they comprise 3 or more carbons, may be linear, branched or cyclic, and wherein several or all hydrogens in said substituent may be replaced by halogen.

According to an embodiment, any one of R³⁰, R³¹, R³² and R³³, in as far as present, is independently selected from C1 to C4, more preferably C1 to C3 and even more preferably C1 to C2 alkyl. Most preferably, any one of R³⁰, R³¹, R³² and R³³, are methyl. Again, said alkyl may be completely or partially halogenated.

Regarding B, it is referred, for example to WO 2014/020499, in particular, page 12, line 22 through page 14, line 31, wherein substituents R¹, R², R³ and R⁴ in WO 2014/020499 are defined as in pages 14, line 27 through page 16, line 8. Preferably, R¹, R², R³ and R⁴ in WO 2014/020499 are selected from substituents R³⁰, R³¹, R³² and R³³, in as far as present, as defined above, also in the context of the bivalent cation B. This disclosure is expressly incorporated herein by reference.

The organic inorganic perovskite layer may be deposited by various techniques for example as disclosed in the international patent application PCT/EP2014/05912, claiming priority of EP13166720.6. In particularly, by sequential deposition, (J. Burschka, N. Pellet, S.-J. Moon, R. Humphry-Baker, P. Gao, M. K. Nazeeruddin, M. Grätzel. Nature 2013, 499, 316).

According to an embodiment, the solar cell of the invention comprises a surface-increasing structure and/or layer. **Figure 8** illustrates a solar cell comprising a surface increasing structure 9. The remaining reference numerals are as disclosed with respect to Fig. 7.

According to a preferred embodiment, the surface-increasing structure comprises or consists essentially of one selected from the group of: a semiconductor material and an insulator material. If the surface-increasing structure comprises a semiconductor material, it is preferably an n-type semiconductor material. Traditionally, the surface increasing structure is fabricated from n-type semiconductor nanoparticles, such as TiO₂ nanoparticles.

Surface increasing structures made from non-conducting materials have also been disclosed. For example, the surface-increasing structure may be made from an insulating material. In this case, the absorber 3, for example the organic inorganic perovskite layer 3, which is deposited on the surface increasing structure 9, is also in contact with an n-type semiconductor layer 2. In this case, the surface increasing structure 9, which is deposited on the n-type semiconductor layer 2 does thus not continuously cover that n-type semiconductor layer 2, so that the absorber 3 can get in contact with the n-type semiconductor layer 2, too. For example, the n-type semiconductor layer 2 may comprise a dense (also known as "compact") n-type nanoparticle layer, and the surface increasing structure 9 is prepared from nanoparticles of the same n-type semiconductor material as the layer 2.

The surface-increasing structure is preferably structured on a nanoscale. The structures of said surface increasing structure increase the effective surface compared to the surface of the solar cell. Preferably, the surface-increasing structure is mesoporous.

The surface-increasing structure is also known as "scaffold structure" or as "surface-increasing scaffold", for example.

According to an embodiment, the surface-increasing structure of the solar cell of the invention comprises and/or consists of nanoparticles. The expression "nanoparticles" encompasses particles or particulate elements, which may have any form, in particular also so-called nanosheets, nanocolumns and/or nanotubes, for example. Nanosheets made from anatase TiO₂ have been reported by Etgar et al., Adv. Mater. 2012, 24, 2202-2206, for example. Preferably, the nanoparticles comprise or consist essentially of said semiconductor material.

The nanoparticles preferably have average dimensions and/or sizes in the range of 2 to 300 nm, preferably 3 to 200 nm, even more preferably 4 to 150 nm, and most preferably 5 to 100 nm. "Dimension" or "size" with respect to the nanoparticles means here extensions in any direction of space, preferably the average maximum extension of the nanoparticles. In case of substantially spherical or ellipsoid particles, the average diameter is preferably referred to. In case of, nanosheets, the indicated dimensions refer to the length and thickness. Preferably, the size of the nanoparticles is determined by transmission electron microscopy (TEM) and selected area electron diffraction (SAED) as disclosed by Etgar et al., Adv. Mater. 2012, 24, 2202-2206.

According to an embodiment, the surface-increasing structure comprises, consists essentially of or consists of one or more selected from the group consisting of Si and metal oxide, including transition metal oxides. For example, the surface-increasing structure comprises a material selected from SiO₂, TiO₂, Al₂O₃, ZrO₂, HfO₂, SnO₂, Fe₂O₃, ZnO, WO₃, Nb₂O₅, In₂O₃, Bi₂O₃, Y₂O₃, Pr₂O₃, CeO₂ and other rare earth metal oxides, CdS, ZnS, PbS, Bi₂S₃, CdSe, CdTe, MgTiO₃, SrTiO₃, BaTiO₃, Al₂TiO₅, Bi₄Ti₃O₁₂ and other titanates, CaSnO₃, SrSnO₃, BaSnO₃, Bi₂Sn₃O₉, Zn₂SnO₄, ZnSnO₃ and other stannates, CaZrO₃, SrZrO₃, BaZrO₃, Bi₄Zr₃O₁₂ and other zirconates, combinations of two or more of the aforementioned and other multi-element oxides containing at least two of alkaline metal, alkaline earth metal elements, Al, Ga, In, Si, Ge, Sn, Pb, Sb, Bi, Sc, Y, La or any other lanthanide, Ti, Zr, Hf, Nb, Ta, Mo, W, Ni or Cu.

According to a preferred embodiment, the surface-increasing structure comprises one or more selected from TiO₂, Al₂O₃, SnO₂, ZnO, Nb₂O₅ and SrTiO₃, for example.

According to an embodiment, said surface-increasing structure forms a continuous and/or complete, or, alternatively, a non-continuous and/or non-complete layer. According to an embodiment, said surface increasing structure forms a layer having an overall thickness of 10 to 3000 nm, preferably 12 to 2000 nm, preferably 15 to 1000 nm, more preferably 20 to 500 nm, still more preferably 50 to 400 nm and most preferably 100 to 300 nm.

For the purpose of the present specification, a "continuous layer" or a "complete layer" is a layer that covers an adjacent layer, such as the conductive support layer, completely so that there can be no physical contact between the two layers separated by the continuous or complete layer and adjacent to said continuous or complete layer. For example, one, two or all selected from the groups consisting of the perovskite layer, the n-type semiconductor layer and the hole transport layer are preferably continuous layers. Preferably, the underlayer, if present, is also a complete layer. The current collector and/or the conductive support are preferably also continuous layers. If the surface increasing structure is non-continuously and/or non-completely provided on said conductive support layer, the perovskite layer does or could get in direct contact with said current collector and/or underlayer.

The surface increasing structure may be prepared by screen printing, spin coating, slot die coating, blade coating, dip coating or meniscus coating, or physical vapor deposition process, for example as is conventional for the preparation of porous semiconductor (e.g. TiO₂) surfaces in heterojunction solar cells, see for example, Noh et al., Nano Lett. 2013, 7, 486-491 or Etgar et al., Adv. Mater. 2012, 24, 2202-2206. The preparation of nanoporous semiconductor structures and surfaces have been disclosed, for example, in EP 0333641 and EP 0606453.

If the surface increasing structure is made of an n-type semiconductor material, the working electrode and/or photoanode of the solar cell of the invention is the assembly of the light-harvesting perovskite and the surface increasing semiconductor material.

In other embodiments, a surface increasing structure as defined above is absent.

The n-type semiconductor layer 2 can be made from n-type semiconductor materials listed above with respect to the surface increasing structure 9. Instead of metal oxides as discussed above, n-type semiconducting polymers may be used, for example, in particular in polymer-based solar cells. This possibility applies in particular if a surface increasing structure made from metal-oxide nanoparticles is absent. Accordingly, layer 2 in Fig. 7, for example, may be an n-type organic material layer as electron transport material (ETM). Organic ETMs can be chosen from a wide variety of conducting materials for charge collection. Exemplary organic ETMs are fullerene and PCBM (Phenyl-C61-butyric-acid-methyl-ester).

According to an embodiment, the solar cell of the invention preferably comprises a current collector. The current collector preferably forms a continuous layer and is preferably adapted to collect the current (and/or electrons) generated by the solar cell and to carry it to an external circuit. The current collector preferably provides the electric front contact of the solar cell.

The current collector preferably comprises a conducting or semiconducting material, such as a conducting organic or inorganic material, such as a metal, doped metal, a conducting metal oxide or doped metal oxide, for example. In a preferred embodiment, the current collector comprises a transparent conductive oxide (TCO). In some preferred embodiments, the current collector comprises a material selected from indium doped tin oxide (ITO), fluorine doped tin oxide (FTO), ZnO-Ga₂O₃, ZnO-Al₂O₃, tin oxide, antimony doped tin oxide (ATO), SrGeO₃ and zinc oxide, or combinations thereof.

The current collector is preferably arranged to collect and conduct the current generated in the working electrode or photoanode. Therefore, the current collector is preferably in electric contact with the working electrode or photoanode. Preferably, the current collector 5 is in direct contact with the n-type semiconductor layer 2.

According to an embodiment, the solar cell of the invention preferably comprises one or more support layers. The support layer preferably provides the physical support of the device. Furthermore, the support layer preferably provides a protection with respect to physical damage and thus delimits the solar cell with respect to the outside, for example on at least one of the two major sides of the solar cell. The support layer is not specifically shown in the figures. In some embodiments, glass or plastic coated with a TCO current collector is used. These materials, which are commercially available, contain the support as well as the current collector. For example, layer 5 in Figs. 7 and 8 can be regarded as a TCO-coated glass or plastic, wherein the TCO faces layer 2 and the glass or plastic faces the outside of the cell and provide the outer surface or side 7.

As shown in Figs. 7 and 8, the solar cell of the invention preferably comprises a conducting current and/or electron providing layer 6. This layer can also be regarded as the counter electrode, which preferably comprises a material that is suitable to provide electrons and/or fill holes towards the inside of the device. In particular, the layer 6 is preferably provided on the HTM layer and injects electrons into the holes that have moved from the perovskite layer 3 across the HTM layer 3. Layer 6 is preferably connected to the external circuit, for example, thereby providing the electric back contact of the solar cell, required for providing electrons that were removed during operation of the solar cell at the current collector layer 7. In this regard, the solar cell of the invention is preferably a regenerative device. The conducting current and/or electron providing layer 6 may, for example, comprise one or more materials selected from (the group consisting of) Pt, Au, Ni, Cu, Ag, In, Ru, Pd, Rh, Ir, Os, C, MoO, including carbon nanotubes, graphene and graphene oxide and a combination of two or more of the aforementioned.

The conducting current or electron providing layer may be applied as is conventional, for example by thermal or electron beam evaporation, sputtering, printing (inkjet printing or screen printing) or spraying process, optionally dispersed or dissolved in solvent-based carrier medium, onto the hole transport layer. The process for applying the counter electrode preferably depends on the material chosen. If an organic material is selected for the counter electrode, such as a carbon based electrode, it can be deposited by inkjet or screen printing. Metals like Ag or Cu can deposited as a paste.

The solar cell of the invention may comprise additional layers, such as blocking layers, additional perovskite layers, support layers, and so forth, as known in the art.

Appropriate coating techniques with respect to the different layers of the device of the invention have been disclosed. It is noted that the deposition of the layers may start from either side: i.e. anode or cathode electrode. When the device comprises a mesoscopic, surface increasing structure, the preparation may start with the deposition of the n-type semiconductor layer on the current collector, as the metal-oxide based nanoporous surface increasing structure generally requires sintering. Components that are more sensitive to heat are then deposited in later steps. In inverted devices, deposition generally starts from the side of the cathode, with the deposition of the HTM layer 4 on the conductive electron providing layer 6 (Fig. 7), followed by the deposition of the perovskite layer 3 and of a n-type semiconductor layer 2, before applying the current collector layer 5.

In a preferred embodiment, the synthesis of the compounds of the invention starts on the basis of the commercially obtainable triazatruxene basic structure (no. (1) in Fig. 1), in which hydrogens are substituted in accordance with the exact compound of the invention to be obtained. Preferably, hydrogens at nitrogen atoms are substituted first, so as to provide substituents R¹. In a second, optional step, hydrogens of carbon atoms may be substituted so as to provide optional substituents R²-R⁵ other than hydrogen. In a preferred embodiment, the hydrogen at R³ is substituted, wherein R², R⁴ and R⁵ remain hydrogens.

In an embodiment, substituents R¹ are introduced so as to increase the solubility of the compounds, and substituents R²-R⁵ introduced are selected so as to adjust the redox potential of the compound, in order to match the required energy level. If such an adjustment is not necessary, R²-R⁵ are preferably hydrogen.

The present invention will now be illustrated by way of examples. These examples do not limit the scope of this invention, which is defined by the appended claims.

### Examples:

### 1. Synthesis of exemplary compounds in accordance with embodiments of the invention

### 1.1 Materials and Methods

All reagents from commercial sources were used without further purification, unless otherwise noted and reactions were performed under dry N₂. All dry reactions were performed with glassware that was flamed under high-vacuum and backfilled with N₂. All extracts were dried over powdered MgSO₄ and solvents removed by rotary evaporation under reduced pressure. Flash chromatography was performed using Silicycle UltraPure SilicaFlash P60, 40-63 µm (230-400 mesh). ¹H (proton) and ¹³C (carbon 13) NMR (nuclear magnetic resonance) spectra were recorded and are reported in ppm using solvent as an internal standard: Deuterated chloroform (CDCl₃) at 7.24 ppm and 77.23 ppm for ¹H and ¹³C, respectively; Deuterated benzene C₆D₆ at 7.16 ppm and 128.39 ppm for ¹H and ¹³C, respectively; Dimethyl Sulfoxide-d₆ at 2.50 ppm and 39.51 ppm for ¹H and ¹³C, respectively.

### 1.2 The basic starting materia/ 10,15-dihydro-5H-diindolo[3,2-a:3',2'-c]carbazole (1).

A mixture of 2-indolinone (10 g, 75 mmol) and POCl₃ (50 mL) was heated at 100 °C for 8h. Then, the reaction mixture was poured into ice and neutralized carefully with NaOH. After neutralization, the precipitate was filtered to give the crude product as a brown solid. The crude was plugged through thick silica-gel pad and recrystallized from acetone resulting pure pale yellow solid having a mass of 3.5g. The yield obtained is: 14 %). The ¹H NMR (400 MHz, DMSO-d₆): δ 11.88 (s, 3H), 8.68 (dd, J = 7.7, 1.4 Hz, 3H), 7.73 (dt, J = 7.9, 1.0 Hz, 3H), 7.36 (dtd, J = 22.3, 7.2, 1.2 Hz, 6H).

The triazatruxene basic structure (1) was used in further experiments as starting material for the synthesis of further intermediates (2), and (3) and/or compounds of the invention HPDI and HMDI in accordance with the scheme shown in **Figure 1****.**

### 1.3 5,10,15-trihexyl-10,15-dihydro-5H-diindolo[3,2-a:3',2'-c]carbazole (2).

To a solution of (1) (400 mg, 1.2 mmol, 1 eq.) in DMF (10 mL), NaH (0.1 g, 4.1 mmol, 3.5 eq.) was added at room temperature and stirred for half hour, then 1-bromohexane (0.76 g, 4.63 mmol, 4 eq.) was added via syringe and the mixture was then refluxed for 2h. The cooled mixture was poured into water and extracted with DCM. The organic phase was dried over MgSO₄. The product was isolated off on a silica gel column with 20 % DCM in hexane to give a product as a pale yellow solid having a mass of 450mg. The yield obtained is 92%). The ¹H NMR (400 MHz, CDCl₃-d): δ 8.32 (d, J = 8.0 Hz, 3H), 7.70 - 7.63 (m, 3H), 7.55 - 7.44 (m, 3H), 7.37 (m, 3H), 4.99 - 4.90 (m, 6H), 2.02 (m, 6H), 1.41 - 1.28 (m, 18H), 0.84 (t, J = 7.0 Hz, 9H).

### 1.4 3,8,13-tribromo-5,10,15-trihexyl-10,15-dihydro-5H-diindolo[3,2-a:3',2'-c]carbazole (3).

To a solution of (2) (350 mg, 0.58 mmol, 1 eq.) in 30 ml CHCl₃, (310 mg, 1.75 mmol, 3 eq.) of NBS in 5 ml DMF was added dropwise via syringe at 0 °C. After addition reaction mixture was stirred for 1h at room temperature. The mixture was extracted with DCM and organic phase was dried over MgSO₄. The product was isolated off on a silica gel column with 10 % DCM in hexane to give a product as a pale-yellow solid having a mass of 400mg. The yield obtained is82%). The ¹H NMR (400 MHz, CDCl₃-d): δ 8.05 (d, J = 8.0 Hz, 3H), 7.71 (s, 3H), 7.55 (d, J = 8.0 Hz, 3H), 4.99 - 4.90 (m, 6H), 2.02 (m, 6H), 1.41 - 1.28 (m, 18H), 0.84 (t, J = 7.0 Hz, 9H).

### 1.5 5,10,15-trihexyl-3,8,13-trimethoxy-10,15-dihydro-5H-diindolo[3,2-a:3',2'-c]carbazole (HMDI).

In a 50 ml three-necked flask, a solution of 1.95 ml (10.5 mmol, 15 eq.) of sodium methoxide 5.4 M in methanol, dry DMF (15 ml), copper(I) iodide (810 mg, 4.3 mmol, 6 eq.), (3) (0.6 g, 0.72 mmol, 1 eq.) was heated to reflux for 3 h under a N₂ atmosphere. After that, solution was filtered while hot through the celite to remove copper(I) iodide and washed with water. The mixture was extracted with DCM and organic phase was dried over MgSO₄. The product was isolated off on a silica gel column with 50 % DCM in hexane to give a product as a pale yellow solid having a mass of 300mg. The yield obtained is 55 %. The ¹H NMR (400 MHz, CDCl₃-d): δ 8.05 (d, J = 8.0 Hz, 3H), 7.71 (s, 3H), 7.55 (d, J = 8.0 Hz, 3H), 4.95 (s, 6H), 4.03 (s, 9H), 2.02 (m, 6H), 1.41 - 1.28 (m, 18H), 0.84 (t, J = 7.0 Hz, 9H); and the ¹³C NMR (100 MHz, Benzene-d₆): δ 157.31, 143.12, 138.04, 122.55, 118.14, 107.27, 104.20, 95.97, 55.02, 46.71, 31.23, 29.28, 26.23, 22.35, 13.71. C₄₅H₅₇N₃O₃[M⁺] Exact Mass = 687.44, MS (mass spectrometri) (ESI) (electrospray ionization) = 687.46.

### 1.6 1-(hexyloxy)-4-iodobenzene (4).

*p*-iodophenol (2 g, 9 mmol, 1 eq.), 1-bromohexane (2.25 g, 13.5 mmol, 1.5 eq.), K₂CO₃ (2.5 g, 18 mmol, 2 eq.) and DMF (20 ml) were placed in a one-neck 100 ml flask equipped with a reflux condenser and a magnetic stir bar. The mixture was refluxed overnight, cooled and poured into water, following by neutralization by NaOH. The resulting mixture was extracted with DCM. The organic mixture was dried over anhydrous MgSO₄. After filtration, the solvent was removed by rotary evaporation. The residue was purified by column chromatography using hexane as the eluent to afford compound as a transparent oil having a mass of 2.5g. The yield obtained is 75 %). The ¹H NMR (400 MHz, CDCl₃-d): δ 7.56 - 7.54(d, J = 9.00 Hz, 2H), 6.69 - 6.67 (d, J = 9.00 Hz, 2H), 3.93 - 3.91 (t, J = 6.50 Hz, 2H), 1.79 -1.76 (m, 2H), 1.47 - 1.44 (m, 2H), 1.38 - 1.36(m, 4H), 0.92 - 0.90 (t, J = 7.00 Hz, 3H).

### 1.7 5,10,15-tris(4-(hexyloxy)phenyl)-10,15-dihydro-5H-diindolo[3,2-a:3',2'-c]carbazole (HMDI).

To a solution of (1) (250 mg, 0.74 mmol, 1 eq.), (4) (0.9 g, 3 mmol, 4 eq.) in 5 ml of quinoline, CuI (550 mg, 2.9 mmol, 4 eq.) and K₂CO₃ (400 mg, 2.9 mmol, 4 eq.) were added. After stirring at 190 °C overnight under a N₂ atmosphere, the reaction mixture was allowed to cool to room temperature and subsequently diluted with DCM followed by filtering through a plug of celite. The filtrate was concentrated in a vacuum. The residue was purified by column chromatography eluting with 30 % DCM in hexane to give 170 mg of pale yellow solid. The yield obtained is 55 %. The ¹H NMR (400 MHz, CDCl₃-d): δ 7.59 (d, J = 6.8 Hz, 6H), 7.30 (d, J = 8.3 Hz, 6H), 7.13 - 7.25 (m, 6H), 6.85 (t, J = 8.0 Hz, 3H), 6.21 (d, J = 8.1 Hz, 3H), 4.14 (t, J = 5.9 Hz, 6H), 2.13 - 1.83 (m, 6H), 1.44 (t, J = 57.8 Hz, 18H), 0.98 (s, 9H); and the ¹³C NMR (100 MHz, C₆D₆-d₆): δ 159.06, 142.55, 138.43, 133.86, 130.08, 127.57, 123.18, 123.07, 120.23, 115.53, 110.28, 104.58, 68.04, 31.58, 29.15, 25.72, 22.67, 13.94. C₆₀H₆₃N₃O₃[M⁺] Exact Mass = 873.4869, MS (MALDI-TOF) (matrix-assisted laser desorption/ionization -time of flight) = 873.1144.

### 2. Fabrication of solar cells in accordance with the invention

### 2.1 Materials and Methods

CH₃NH₃I was synthetized preparing an equimolar solution of methylamine (40% in water) and HI (hydriodic) (57% in water) in an ice bath to control the temperature.

### 2.2 Device fabrication

FTO-coated glass was laser etched. The substrates were cleaned with Hellmanex and rinsed with deionized water and ethanol. After that, the samples were ultrasonicated in 2-propanol and dried with compressed air. Preceding the compact layer deposition, the substrates were UV/O₃ treated to eliminate organic residues.

A blocking TiO₂ layer was deposited by spray pyrolysis using a titanium diisopropoxide bis(acetyl acetonate) solution (1ml of commercial titanium diisopropoxide bis(acetyl acetonate) 75% in 2-propanol in 19ml of pure ethanol) using O₂ as carrier gas. During that process, etched substrates were heated at 450°C to facilitate the anatase formation. After cooling down, substrates were immersed in TiCl₄ 20mM aqueous solution and baked 30minutes at 70°C. Then the samples were washed with deionized water and heated 500°C for 30 minutes. After cooling down, substrates were cut in the proper cell size.

TiO₂ mesoporous layer (mp-TiO₂) was prepared by spin coating 35 µl per cell of a solution made of 1g of 30NR-D in 3.5g of absolute ethanol. The spin coating conditions employed were: speed=4000rpm, acceleration=2000rpm·s⁻¹, time=30s. Subsequently, samples were sequentially sintered: 125°C for 5min, 325°C for 5min, 375°C for 5min, 450°C for 15min and 500°C for 15min.

PbI₂ film was deposited using double coating of PbI₂ solution, 1.25M concentrated in DMF was kept at 70°C to avoid any precipitation. 50µl from that solution were deposited and spun coated over the mesoporous TiO₂ film. Subsequently these films were annealed at 70°C for 30 minutes. After cooling down, the spin coating process is repeated using the same spin-coating parameters. During that, a partial dilution of the first PbI₂ film was observed. The annealing step was repeated as well. 100 µl of CH₃NH₃I solution (8mg·ml⁻¹) were spread above the PbI₂ film to form perovskite waiting 20-25s to see the conversion of PbI₂ into CH₃NH₃PbI₃ (from yellow to black). Finally, the excess of solvent was removed by spin coating. and further annealed at 70°C for 30minutes.

Hole transporting materials (HTM), either 5,10,15-trihexyl-3,8,13-trimethoxy-10,15-dihydro-5H-diindolo[3,2-a:3',2'-c]carbazole **(HMDI)** or 5,10,15-tris(4-(hexyloxy)phenyl)-10,15-dihydro-5H-diindolo[3,2-a:3',2'-c]carbazole **(HPDI),** depending on the device, were spun coated (speed=2000rpm, acceleration=1000rpm·s⁻¹, time=30s). HMDI solution was 0.04241M and HPDI 0.03338M in Chlorobenzene was taken.

In some exemplary devices, solutions of dopants with additives were also included:
In some devices, LiTFSI dopant and t-BP additive were added to HMDI. In these devices, LiTFSI and t-BP were added the following concentrations expressed in mol-percentage with respect to the HMDI: LiTFSI: 25.5 mol.%; and additives: t-BP: 141 mol%.
In some devices containing HMDI, tris(2-(1H-pyrazol-1-yl)-4-tert-butylpyridine)cobalt(III) tris- (bis(trifluoromethylsulfonyl)imide)) (FK 209) dopant was added together with LiTFSI dopant and t-BP additive. In these devices, dopants and additives were present at the following concentrations expressed in mol-percentage with respect to the HMDI, dopants: FK 209: 13.3 mol.%; LiTFSI: 25.5 mol.%; and additives: t-BP: 141 mol%.
In devices containing HPDI, dopants were used at the following concentration, if present: LiTFSI: 32 mol%; and additives were used at the following concentration, if present: t-BP: 179 mol % (both with respect to the molar amount of HPDI).
A cathode composed of gold of 80nm of thickness was used and deposited by thermal evaporation.

### 2.3 Characterization of devices

For J-V curves,were calculated using AM (air mass) 1.5 G as a source. For IPCE measurements, a power source, 300W Xe lamp with a Power supply was used and connected to a monochromator. Results are shown in Table 1 below:

**Table 1: PV performance of solar cells of the invention.**

| Configuration | J_{sc} (mA/cm² ) | V_{oc} (V) | FF | η (%) |
|---|---|---|---|---|
| FTO/bl-TiO₂/mp-TiO₂/CH₃NH₃PbI₃/HMDI/Au | 14.43 | 0.938 | 0.637 | 8.62 |
| FTO/bl-TiO₂/mp-TiO₂/CH₃NH₃PbI₃/ HMDI+tBP+LiTFSI /Au | 13.70 | 0.868 | 0.715 | 8.50 |
| FTO/bl-TiO₂/mp-TiO₂/CH₃NH₃PbI₃/ HMDI+tBP+LiTFSI+FK209/Au | 16.63 | 0.810 | 0.613 | 8.26 |
| FTO/bl-TiO₂/mp-TiO₂/CH₃NH₃PbI₃/HPDI/Au | 17.9543 | 0.894 | 0.641 6 | 10.30 |
| FTO/bl-TiO₂/mp-TiO₂/CH₃NH₃PbI₃/ HPDI+tBP+LiTFSI /Au | 9.97 | 0.901 | 0.650 | 5.84 |

| | | | | |
|---|---|---|---|---|
| FF: Fill Factor; η: efficiency | | | | |

In various devices prepared, power conversion efficiencies of 8-10% were routinely achieved. A particular cell achieved an efficiency of close to 11% (not shown in Table 1). In several devices, in particular those based on HPDI, doping had a negative impact in the performance, and in those based on HMDI, doping and without doping resulted in a similar impact on the performance. In general, doping by LiTFSI and t-BP did not improve PV properties.

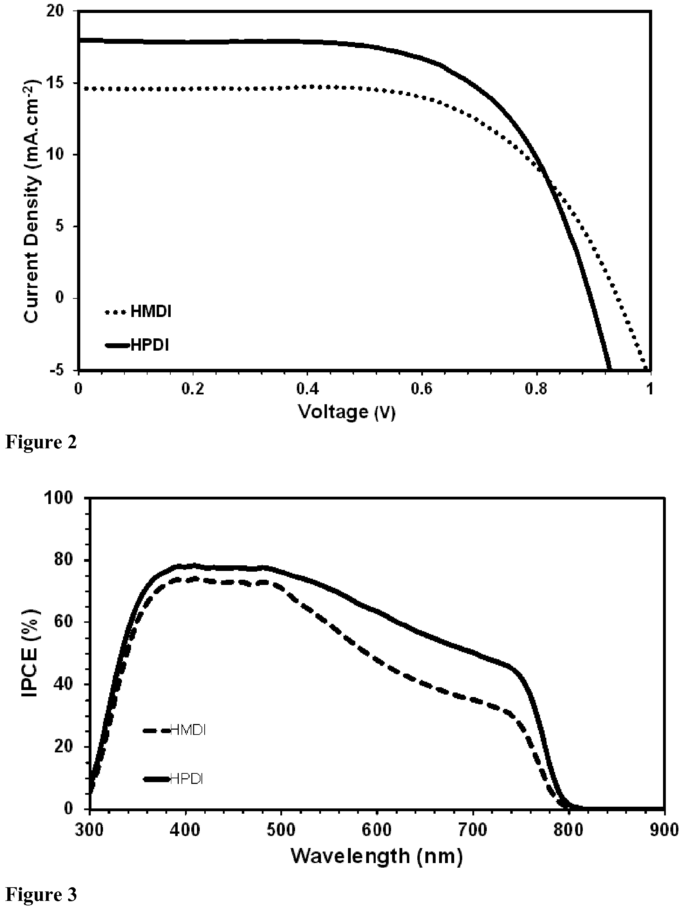

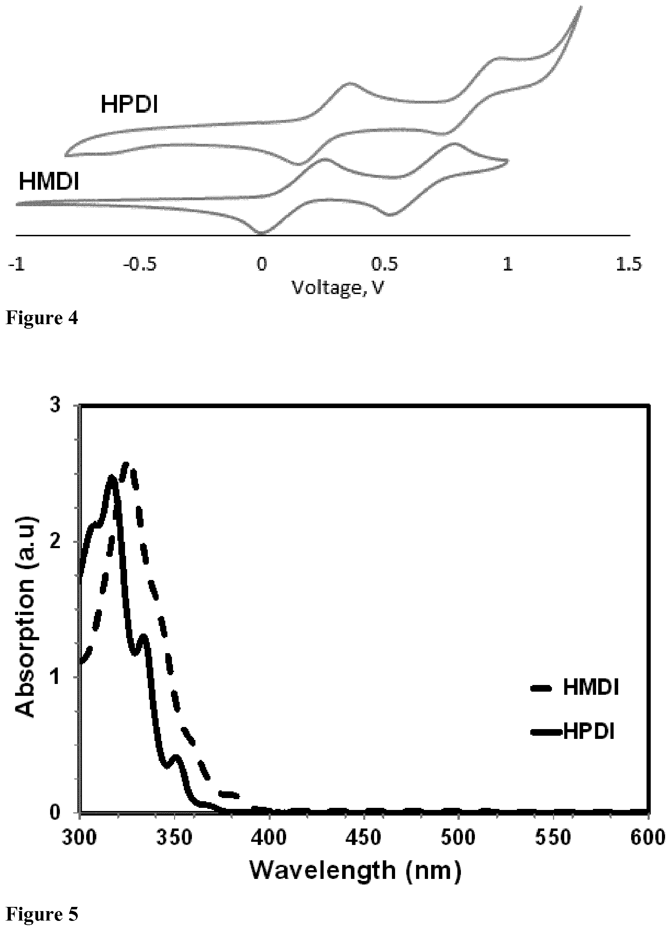

More detailed performance of devices of the invention are shown in **Figures 2** and **3****.** The cyclic voltamograms and the UV-Vis absorption spectra shown in **Figures 4** and **5** show the characterization of the compounds. Both compounds absorbs in the UV region between 250-350 nm. Thermogravimetirc analysis (TGA) curves of the compound HMDI and HPDI as such is shown in **Figure 6****,** of the invention are stable at temperatures of 350 for HPDI and at 400°C for HMDI.

In general, the compounds of the invention are stable at temperatures of 300°C or above, preferably 350°C or above.

Comparisons were also made between devices prepared in accordance with the present invention and devices prepared with Spiro-OMeTAD, the device in solid state that shows highest performance nowadays. Results are shown in Table 2:

**Table 2: Power conversion efficiency of solar cells of the invention with SA (in accordance with the invention) compared to Spiro-OMeTAD.**

| ID | Light intensity [mW cm⁻²] | Direction | V_{OC} [mV] | J_{SC} [mA cm⁻²] | FF | PCE [%] |
|---|---|---|---|---|---|---|
| S4 | 99,3 | FB a SC | 1145 | 20.7 | 0.77 | 18.3 |
| | | SC a FB | 1147 | 20.6 | 0.72 | 17.0 |
| *spiro-*OMeTAD | 97,8 | FB a SC | 1087 | 22.3 | 0.74 | 17.9 |
| | | SC a FB | 1059 | 22.3 | 0.69 | 16.3 |

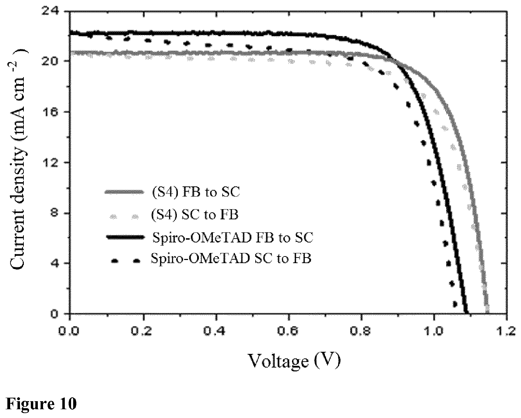

**Figure 10** shows a J-V curve. The curves were recorded scanning at 0.01 V s⁻¹ from forward bias (FB) to short circuit (SC) condition and vice versa. The prepared HTM (S4) showed a lower hysteresis value.

J-V curves for both (S5) and (S6) were calculated using an AM (air mass) of 1.5 G as a source. For the measures of the IPCE an electric source, a 300 W Xe lamp with a power supply were used and were connected to a monochromator. Results are included in table 3 that is shown below:

**Table 3: Power conversion efficiency of solar cells of the present invention.**

| ID | Light intensity [mW cm⁻²] | V_{OC} [mV] | J_{SC} [mA cm⁻²] | FF | PCE, % |
|---|---|---|---|---|---|
| S6 | 98.4 | 964 | 18.9 | 0.70 | 12.9 |
| S5 | 98.4 | 989 | 18.7 | 0.69 | 12.9 |

## Claims

1. A compound comprising the structure of formulae (I) below: 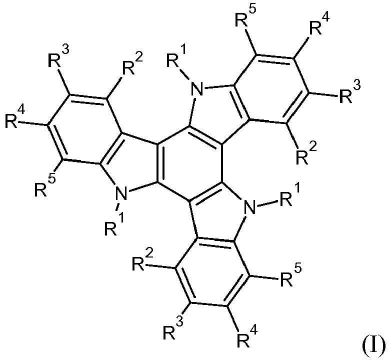 wherein R¹ is selected from substituted or unsubstituted alkyl, alkenyl, alkynyl, and aryl, and wherein R²-R⁵, are selected independently from H, substituted or unsubstituted alkyl, alkenyl, alkynyl, aryl, and substituents of formula (II), (III) and (IV) below, 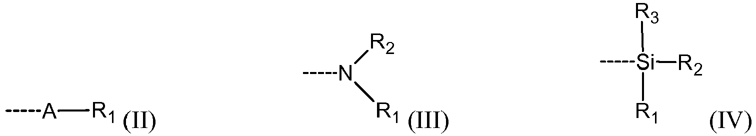 wherein A is selected from O, S, Se, or another electron donor moiety, and R₁, R₂, and R₃, in as far as present, are independently selected from alkyl, alkenyl, alkynyl, aryl; wherein any one of said alkyl, alkenyl, and alkynyl may be linear, branched or cyclic.

2. The compound of claim 1, wherein R¹ is selected from alkyl and substituted aryl, wherein substituents of said aryl are selected from alkyl and from substituents of formula (II), (III) or (IV), and wherein R²-R⁵, are selected independently from H, alkyl, and substituents of formula (II), (III) or (IV).

3. The compound of claim 1 or 2, wherein R¹ is selected from alkyl and substituted phenyl, wherein substituents of said phenyl are selected, independently, from alkyl and from alkoxyl, and wherein R²-R⁵ are selected, independently, from H, alkyl, and alkoxyl.

4. The compound of any one of the preceding claims, which is a compound of formula (V): 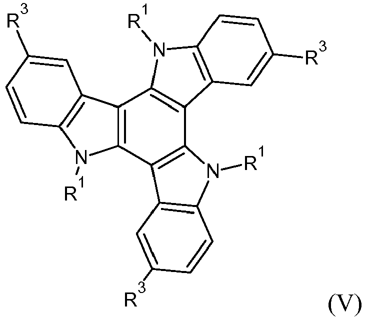 wherein R¹ is as defined in any one of claims 1-3 and wherein R₃ is defined as R²-R⁵ in any one of claims 1-3, respectively.

5. The compound of any one of the preceding claims, which is selected from a compound of formula (VI) or (VII) below: 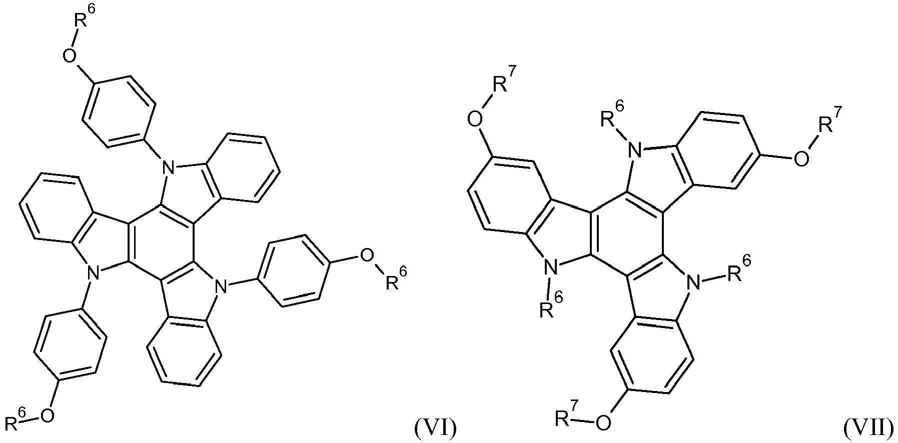 wherein R⁶ and R⁷ are independently selected from a linear, branched or cyclic C1-C12 alkyls.

6. The compounds of claim 5, wherein R⁶ is selected from linear and branched C4-C10 alkyls and R⁷ is selected from linear and branched C1-C10 alkyls.

7. The compound of any one of the preceding claims, which is soluble in any one, several or all solvents selected from the group consisting of: chlorobenzene, benzene, 1,2-dichlorobenzene, toluene and chloroform at more than 50 mg of compound per ml of solvent at 25°C.

8. The compound of claim 7, which is soluble in any one, several or all solvents mentioned in claim 7 at more than 100 mg of compound per ml of solvent at 25°C.

9. The compound of any one of the preceding claims, which is selected from 5,10,15-trihexyl-3,8,13-trimethoxy-10,15-dihydro-5H-diindolo[3,2-a:3',2'-c]carbazole and 5,10,15-tris(4-(hexyloxy)phenyl)-10,15-dihydro-5H-diindolo[3,2-a:3',2'-c]carbazole.

10. An optoelectronic device comprising the compound of any one of claims 1-9.

11. The optoelectronic device of claim 10, which is a solar cell, preferably a solid state solar cell.

12. The optoelectronic device of any one of claims 10-11, which is an organic-inorganic perovskite-sensitized solar cell.

13. The optoelectronic device (1) of any one of claims 10-12, which comprises a conducting current collector layer (5), a n-type semiconductor layer (2), an organic-inorganic perovskite layer (3), a hole transport layer (4) and a conducting current providing layer (6), wherein said hole transport layer (4) is provided between said perovskite layer (3) and said current providing layer (6), said hole transport layer comprising a hole transport material comprising the compound of any one of claims 1-9.

14. The optoelectronic device of any one of claims 10-13, wherein said hole transport layer has a thickness of 50-400 nm, preferably 100-200 nm, even more preferably 110-190 nm or 120-180 nm, and most preferably 130-170 nm or 135-165 nm, 140-160 nm, for example about 150 nm.

15. Use of the compounds of any one of claims 1-9 as a hole transport material (HTM).

16. A process for producing a solar cell (1) comprising the steps of applying a plurality of layers comprising an organic-inorganic perovskite layer (3), a hole transport layer (4) and a conducting current providing layer (6), wherein said hole transport layer (4) comprises an HTM comprising a compound selected from the compounds as defined in any one of claims 1-9.

17. A process for producing the compounds of any one of claims 1-9, comprising the steps of:
(i) substituting the nitrogen atoms of a triazatruxene basic structure (1) by a substituent selected from substituted or unsubstituted alkyl, alkenyl, alkynyl, and aryl (R¹ in any one of claims 1-9); and, optionally,
(ii) substituting one or more hydrogen atoms of benzene rings of the triazatruxene basic structure (1) by substituents selected from the group consisting of: substituted or unsubstituted alkyl, alkenyl, alkynyl, aryl, and substituents of formula (II), (III) and (IV) below, 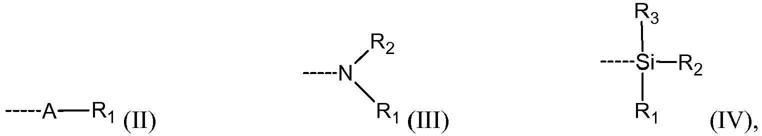 wherein A is selected from O, S, or Se, and R₁, R₂, and R₃, in as far as present, are independently selected from alkyl, alkenyl, alkynyl, aryl;
wherein any one of said alkyl, alkenyl, and alkynyl in step (i) or step (ii) may be linear, branched or cyclic.

18. The process of claim 17, wherein said step (ii) is conducted by halogenating one or more hydrogen atoms of benzene rings of the triazatruxene basic structure (1) and by substituting halogen atoms by said substituted or unsubstituted alkyl, alkenyl, alkynyl, aryl, and substituents of formula (II), (III) and (IV).
